# EUROPEAN PATENT SPECIFICATION

(11) **EP 1 296 696 B1**
(45) Date of publication and mention of the grant of the patent: **28.02.2007**
(21) Application number: 00934835.0
(22) Date of filing: 19.06.2000
(51) Int. Cl.: A61K 36/28, A61K 36/61, A61P 31/00

(54) **HERBAL COMPOSITION CONTAINING CALENDULA AND EUCALYPTUS FOR THE TREATMENT AND CURING OF DISEASES**
KRÄUTERZUSAMMENSETZUNG ENTHALTEND CALENDULA UND EUCALYPTUS FÜR DIE BEHANDLUNG UND HEILUNG VON ERKRANKUNGEN
COMPOSITION A BASE D'HERBES CONTENANT CALENDULA ET EUCALYPTUS DESTINEE AU TRAITEMENT DE MALADIES

(43) Date of publication of application: 02.04.2003
(73) Proprietor: Skopichenko Dettwiler, Victoria, 8049 Zürich (CH); Doujenkova, Svetlana, Moscow, 103 064 (RU)
(72) Inventor: DOUJENKOVA, Svetlana, Moscow, 103 064 (RU)
(74) Representative: Felber, Josef
(86) International application number: PCT/CH2000/000331
(87) International publication number: WO 2001/097823

(56) References cited:
- WO-A-98/42188
- DATABASE WPI Section Ch, Week 199434 Derwent Publications Ltd., London, GB; Class B04, AN 1994-277491 XP002164800 & RU 2 008 013 C (TRESKUNOV K A), 28 February 1994 (1994-02-28)
- DATABASE WPI Section Ch, Week 199221 Derwent Publications Ltd., London, GB; Class B04, AN 1992-174224 XP002164801 & SU 1 659 054 A (MEDICINAL PLANTS RES INST), 30 June 1991 (1991-06-30)

## Description

This invention concerns a composition of natural herbs for the treatment and curing of various diseases in the field of the microbial and viral etiology. For its use, this herbal composition is applied in the form of a tincture or syrup or in suspension form, as a tea, powder as tablets, capsules, analcoholic or alcoholic drinks or as an ointment. It can also be delivered through any other known carriers such as biscuits or snacks and even on the base of other medical herbs which are recommended for the curing of diseases of microbial or viral etiology. The composition is particularly effective for the treatment of HIV-infections (AIDS), Tuberculosis, Infectious Hepatitis and other diseases of the same etiology. For this purpose, the composition always contains the two following basic components:
A: Folium or Tincture of Calendulae
B: Folium or Tincture Eucalypti
For other special applications, the composition may be varied by adding one or more of the following substances:
C: Succus Plantaginis
D: Extractum Aloes fluidum
E: Sirupus fructus Rosae vitaminisatus

These various, easy to use compositions allow a quick and effective method for healing people whereby no lateral adverse side effects occur. Moreover, the products based on this composition are not cost-demanding. The proposed compositions are natural medications which have been tested exhaustively and have proved to have surprisingly effective antimicrobic and antiviral properties in a very ample spectrum of applications. They not only show no negative lateral effects but contain very important vitamins and micro-elements to strengthen the defensive or immune system of an individual against in essence any kind of other infection by tuning up the desintoxication properties of the patient.

So far there is no medication with total antiviral properties to cure people radically without the elimination of virus. The treatments according to the state of the medical art are merely antivirus vaccinations which firstly have the limitations of a pure prophylactic nature and secondarily which frequently lead to complications. This demonstrates that such vaccinations are not completely harmless.

In contradiction thereto, the treatments with the proposed compositions result in a very quick and stable healing. Even within a short time after the application, that is after 1 to 3 days, evidence of a lack of intoxication occurs, further normalized body temperature and the disappearance of pain in muscular and articulations.

Microbicides used today are antibiotics or chemopreparations which sometimes show very quick results but unfortunately are accompanied very often with adverse side effects. They can only be used for a short time, because they tend to be toxic, causing disbacteriosis and have negative influence on the liver, intestine tract or the central nerve system, and the general condition of the individual. The cost factor of such antibiotics and chemopreparations is such that they are presently not affordable in less favoured countries which potentially have even more sick people.

The proposed medication has demonstrated to be highly effective in the healing of Tuberculosis (TBC) in its primary and secondary stadium. Using it against these diseases, after only 20 to 30 days of treatment, dramatic improvements became evident, normalising the blood sedimentation and its spectrum. Coughing disappears completely, perspiration and weakness is overcome, and the general condition of the patient is improved. Experiences show that a complete curing takes 3 to 4 months. The medication has also demonstrated effectiveness in the treatment of Tuberculosis-Meningitis and Meningitis. Due to the alcohol contained in the proposed medication, the absorption of it is much faster, resulting in a better, strong and quicker effect in the central nerve system.

The medication finds also application in oncological treatments for the improvement of general condition and, most importantly, eliminates the very strong pain that characterizes such diseases, avoiding the application of very strong narcotics used in such cases, and also alleviating the heavy financial cost of such treatments. Since all components are totally harmless, they can be taken as a ointment to reinforce the entire organism against virus, microbia, etc., by both children or adults, without interfering with most other medications.

More particular, this invention provides a highly potent herbal composition for the treatment and curing of all diseases of virus etiology such as:
1. HIV-infections, AIDS, Virus Immunodeficiency, Viral Hepatitis, Influenza, Measles, etc.;
2. For the treatment of diseases of microbic etiology like Tuberculosis, Meningitis, Neumonia, etc.;
3. For the treatment of Bronchial Asthma in infecto allergic form;
4. Wound infections through accidents or burns;
5. As post operation antiphlogistic;
6. Against disturbances of the immune system and against low resistance to infections;
7. For the prophylaxis of contagious diseases to third persons;
8. As a pain inhibitor and improvement of general condition of oncological patients;
9. As an alternative to antibiotics and other chemo-medical preparations producing secondary effects like allergies, etc.

In the following, the main healing properties of the used single components as well as these components itself are described and commented as they are known in the state of the medical art:

Component A: **Leaves or Tincture of Calendula *(Folium Calendulae or Tinctura Calendulae)***
In the case of a tincture, this is an alcohol (70% alcohol) tincture with (1:10) of Calendula flowers and flower calathides (Calendula officinalis L.), thistle family (Compositae) in the form of a clear liquid of yellow color, produced and available in vials of 40ml. It has microbicide and antivirial properties and is used in the following cases: Gall bladder problems (for getting relief), cuts, purulent wounds, bums, for throat wash in case of inflammatory diseases of the upper respiratory tract, Angina, etc. For its application, 1 teaspoon is diluted in a glass of water. It can be taken perorally as cholagogue (10-20 drops per taking). Storage: in cool, dark places.

Component B: **Leaves or Tincture of Eucalyptus *(Folium Eucalypti or Tinctura Eucalypti)***
In the case of a tincture, this is a tincture (1:5) based on 70% of alcohol in the form of a clear liquid of greenish brown color with peculiar flavor and is administrated as antiinflammatory and antiseptic preparation in cases of inflammatory diseases of the upper respiratory tract and the mouth cavity, sometimes as a sedative with antiviral and microbicide properties. The dosage for peroral taking is 15-30 drops per dose and for throat washing 10-15 drops per glass of water.

Component C: **Sap of Plantain (*Succus Plantaginis)***
This is a mixture of large plantain fresh leaves sap or succus and plantain bloshiny (Plantago Psyllum L.) above ground part succus. It is preserved with alcohol and sodium metabisulfite (0,15%) and is produced and available in vials of 250ml. It appears as dark, slightly nebulous liquid with foxy color, acidulous to the taste with peculiar flavor. It is used mainly as bitter substance in case of anacidic gastritis and chronic colitis. As microbicide and immune system stimulator, it strengthens the entire organism. Dosage: One table spoon 3 times per day, 15-30 minutes prior to meals. The treatment duration is in average 30 days.

Component D: **Liquid Aloe extract *(Extractum Aloes fluidum)***
This is a water extract of cut canned leaves of Aloe for peroral taking. It is a clear liquid from canary to flame color with bitter taste and is produced and available in vials of 100ml. The indications for its use are the same as in case of Liquid Aloe Extract for injections. It works as microbicide and appetite stimulator. Dosage: 1 tea spoon 3 times per day. Duration of treatment: 30-45 days. 3-4 treatment courses per year are recommended.

Component E: **Vitaminized Rose Hip Syrup *(Sirupus fructus Rosae vitaminisatus)***
This syrup is produced from rose hips. 1ml contains 30mg of ascorbic acid (Vitamin C) and 15mg of vitamin P. The syrup is produced and available in glass cans of 200 or 250ml capacity. The storage temperature must be less than +15°C. It is administered for disease prevention since it has general stimulation properties through a high content of vitamines which also leads to a taste improvement. Dosage for prevention: Adults: 1 tea spoon per day, children: ½ tea spoon per day. Dosage for treatment: 2-3 times per day the prevention dosis.

The main properties of the present herbal composition for the treatment and curing of diseases are:
1. Notorious antiviral properties;
2. Notorious microbicide of wide spectrum;
3. Improvement of liver and gall bladder function;
4. Positive influence on the functioning of the stomach and intestine tract. Stabilization of the intestinal acidity and microflora;
5. Tranquilizer properties for the central nerve system;
6. Normalizing blood pressure;
7. Strengthening and stimulating the immune system;
8. Anaesthetic, calming and sedative properties.

The above mentioned highly potential herbal composition in the different forms can be used in the amounts as indicated in the examples below which are named as Antivirin A, B and C:

Antivirin A:
1. Tincture of Calendula, about 100 to 600 ml
2. Tincture of Eucalyptus, about 50 to 200 ml
3. Succus of Plantain, about 50 to 400 ml
4. Liquid Aloe extract, about 50 to 600 ml
Balance ingredients, if any, are being taken from known additives selected from stabilizers, anti-caking agents, flavoring agents, sweetening agents, thickening agents, preservatives and coloring agents. This substance Antivirin A does not contain sugar and is therefore also suitable for diabetics. Antivirin A is designed for oral application: 1 teaspoon (5ml) dissolved in 50ml of warm water or fruit juice, to be taken 30 minutes before the meals, 3 times per day. In acute cases 2 teaspoons (10m1) in 50 ml of warm water or fruit juice, 6 times per day. Period of application: Unlimited repetitions, until complete clinical recovery. If necessary, Antivirin A can be used even permanently.

Antivirin B:
1. Tincture of Calendula, about 50 to 300 ml
2. Tincture of Eucalyptus, about 20 to 100 ml
3. Succus of Plantain, about 20 to 200 ml
4. Liquid Aloe extract, about 20 to 300 ml
5. Vitaminized Rose hip Syrup, about 200 to 600 ml
Balance ingredients, if any, are being taken from known additives selected from stabilizers, anti-caking agents, flavoring agents, sweetening agents, thickening agents, preservatives and coloring agents. This substance Antivirin B does contain sugar and is therefore not suitable for diabetics. Antivirin B is designed for oral application: 2 tea spoons (10ml) dissolved in 100 ml of warm water, to be taken 30 minutes before the meals, 3 times per day. Period of application: Unlimited repetitions, until complete clinical recovery. If necessary, Antivirin B can be used even permanently.

Antivirin C:
In this execution, the Antivirin herbs extract is prepared as a dry powder in the following composition:
1. Calendula extract, about 20 to 80 gr.
2. Folium Eucalyptus, about 20 to 80 gr.
3. Folium Plantain, about 10 to 50 gr.
Balance ingredients, if any, are being taken from known additives selected from stabilizers, anti caking agents, flavoring agents, sweetening agents, thickening agents, preservatives and coloring agents. For its practical application, Antivirin C is designed for oral application and is being taken as a tea, that is without alcohol or sugar: 1 paper filter bag (5gr.) or soup spoon of the dry product disolved for 15 minutes in 200ml of boiling water in a china or glass container (with cover). Straining into a cup or jar. Dring half a glass (100ml) or in heavy cases a complete glass (200 ml) in warm condition and this 3 times per day, before meals. It can be taken with honey or other sweetening agents. Period of application: Unlimited repetitions, until complete clinical recovery. If necessary, Antivirin C can be used even permanently.

Basically, all these compositions might be prepared as a powder, as tablets, capsules or as a syrup or in suspension form, as an alcoholic or hydroalcoholic extract. The dosis of these medications have been established by extensive practical applications and are such that they ensure maximal curing effects for the above mentioned diseases. The changing of the dosis or components of the medication would change drastically the curing effects! In certain cases, augmenting the dosis of the medication or changing of the components can even be very contraproductive!

For medical personnel or other people in contact with infected patients, Antivirin A, B or C may be taken as prophylactic measure in order to avoid infections. In such cases, it is recommended to take Antivirin A 2 to 3 times per day, up to 10 days after the end of the contact, or Antivirin B, 1 soup spoon (20ml) 2 to 3 times per day, for the same period of time, or Antivirin C, half a glass (100ml) 2 to 3 times per day, up to 10 days after the end of contact.

Patients who have contraindications for sugar or alcohol should only use Antivirin C in the above mentioned dosis. Children under 15 years of age should use Antivirin C in the dosis corresponding to their age. Antivirin A and B should be given to children under 15 years of age only in extreme cases such as e.g. Meningitis. It should then be applied for the necessary period of time as prescribed by the medical doctor. The dosages can be augmented, depending upon the severity of the disease or as prescribed by the medical doctor.

These are only three specific examples of Antivirin (Antiviral A, B, and C), but there are other combinations and dosis of the relevant components which are highly effective. In the following, further combinations are disclosed as basic and specific examples:

Example 1 with basic combination of

| | |
|---|---|
| Tincture of Calendula: | about 40 to 60 ml |
| Tincture of Eucalyptus: | about 10 to 30 ml |
| Succus of Plantain: | about 20 to 40 ml |

Specifically suggested combination to be taken 3 times per day in the amount of 1 to 2 teaspoons:

| | |
|---|---|
| Tincture of Calendula: | 40ml |
| Tincture of Eucalyptus: | 20 ml |
| Succus of Plantain: | 40 ml |

Example 2 with basic combination of

| | |
|---|---|
| Tincture of Calendula: | about 40 to 60 ml |
| Tincture of Eucalyptus: | about 10 to 30 ml |
| Liquid Aloe Extract: | about 20 to 40 ml |

Specifically suggested combination to be taken 3 times per day in the amount of 1 to 2 teaspoons:

| | |
|---|---|
| Tincture of Calendula: | 40 ml |
| Tincture of Eucalyptus: | 20 ml |
| Liquid Aloe Extract: | 40 ml |

Example 3 with basic combination of

| | |
|---|---|
| Tincture of Calendula: | about 40 to 60 ml |
| Tincture of Eucalyptus: | about 10 to 30 ml |
| Vitamin Rose hip syrup: | about 40 to 60 ml |

Specifically suggested combination to be taken 3 times per day in the amount of 1 to 2 teaspoons:

| | |
|---|---|
| Tincture of Calendula: | 40 ml |
| Tincture of Eucalyptus: | 20 ml |
| Vitamin Rose hip syrup: | 40 ml |

Example 4 with basic combination of

| | |
|---|---|
| Tincture of Calendula: | about 40 to 60 ml |
| Tincture of Eucalyptus: | about 10 to 30 ml |
| Succus of Plantain: | about 10 to 20 ml |
| Vitamin Rose hip syrup: | about 40 to 60 ml |

Specifically suggested combination to be taken 3 times per day in the amount of 2 teaspoons up to 3 soup spoons:

| | |
|---|---|
| Tincture of Calendula: | 20 ml |
| Tincture of Eucalyptus: | 10 ml |
| Succus of Plantain: | 20 ml |
| Vitamin Rose hip syrup: | 50 ml |

Example 5 with basic combination of

| | |
|---|---|
| Tincture of Calendula: | about 40 to 60 ml |
| Tincture of Eucalyptus: | about 10 to 30 ml |
| Liquid Aloe extract: | about 20 to 40 ml |
| Vitamin Rose hip syrup: | about 40 to 60 ml |

Specifically suggested combination to be taken 3 times per day in the amount of 2 teaspoons up to 3 soup spoons:

| | |
|---|---|
| Tincture of Calendula: | 20 ml |
| Tincture of Eucalyptus: | 10 ml |
| Liquid Aloe extract: | 20 ml |
| Vitamin Rose hip syrup: | 50 ml |

The herbal composition can be used in still other compositions. For example for the treatment or curing of diseases such as Virus lmmunodefficiency, HIV-infections, AIDS, Viral Hepatitis, TBC, Meningitis and other virus or microbial etiology diseases in children and adults, a herbal composition containing both Tincture of Calendula and Tincture of Eucalyptus is useful if it contains 100ml of composition in hydro-alcoholic suspension, and containing the substances in the following ratio: 50 ml of Tincture of Calendula and 50ml of Tincture of Eucalyptus.

The Herbal composition as presented can also be used in forms where it contains additives such as stabilizers, anti-caking agents, thickening agents, preservatives and coloring agents.

## Claims

1. Herbal composition for use in the treatment or curing of diseases such as Virus Immunodefficiency, HIV-infections, AIDS, Viral Hepatitis, TBC, Meningitis and other virus or microbial etiology diseases in children and adults, containing at least Folium or Tincture of Calendula and Folium or Tincture of Eucalyptus.

2. Herbal composition according to claim 1, containing in addition Sucus of Plantain.

3. Herbal composition according to one of the preceding claims, containing in addition Liquid Aloe extract.

4. Herbal composition according to one of the preceding claims, containing in addition Vitaminized Rose Hip Syrup.

5. Herbal composition according to claim 3, containing the substances in the following ratio:
| | |
|---|---|
| Tincture of Calendula | 100 to 600ml |
| Tincture of Eucalyptus | 50 to 200ml |
| Sucus of Plantain | 50 to 400ml |
| Liquid Aloe extract | 200 to 600ml. |

6. Herbal composition according to claim 4, containing the substances in the following ratio:
| | |
|---|---|
| Tincture of Calendula | 100 to 600ml |
| Tincture of Eucalyptus | 50 to 200ml |
| Sucus of Plantain | 50 to 400ml |
| Liquid Aloe extract | 50 to 600ml |
| Vitaminized Rose hip syrup | 200 to 600ml. |

7. Herbal composition according to claim 2 in powder or paste form, containing the substances in the following ratio:
| | |
|---|---|
| Flowers of Calendula | 50gr. |
| Folium of Eucalyptus | 25gr. |
| Folium of Plantain | 25gr. |

8. Herbal composition according to claim 1, wherein 100ml of composition in alcoholic extract form contains the substances in the following ratio:
| | |
|---|---|
| Tincture of Calendula | 70ml |
| Tincture of Eucalyptus | 30ml. |

9. Herbal composition according to claim 1, wherein 100ml of composition in hydro-alcoholic suspension contains the substances in the following ratio:
| | |
|---|---|
| Tincture of Calendula | 50ml |
| Tincture of Eucalyptus | 50ml. |

10. Herbal composition according to one of the preceding claims, containing additives selected from stabilizers, anti-caking agents, thickening agents, preservatives and coloring agents.

## Patentansprüche

1. Kräutermischung für die Behandlung oder das Heilen von Krankheiten wie Virusimmundefizite, HIV-Infektion, AIDS, Virale Hepatitis, Tuberkulose (TBC), Meningitis und andere Virus- oder Mikrobenätiologiekrankheiten bei Kindern und Erwachsenen, wobei die Mischung mindestens das Folium oder eine Tinktur der Calendula und das Folium oder eine Tinktur des Eukalyptus enthält.

2. Kräutermischung nach Anspruch 1, ***dadurch gekennzeichnet,* dass** die Mischung zusätzlich den Succus der Plantago Major (Wegerich) enthält.

3. Kräutermischung nach einem der vorangehenden Ansprüche, ***dadurch gekennzeichnet,* dass** die Mischung zusätzlich flüssiges Aloeextrakt enthält.

4. Kräutermischung nach einem der vorangehenden Ansprüche, ***dadurch gekennzeichnet,* dass** die Mischung zusätzlich vitaminisierten Hagebutte-Sirup enthält.

5. Kräutermischung nach Anspruch 3, **dadurch gekennzeichnet, dass** sich die Mischung aus folgenden Anteilen zusammensetzt:
| | |
|---|---|
| Calendula-Tinktur | 100 bis 600ml |
| Eukalyptus-Tinktur | 50 bis 200ml |
| Succus der Plantago Major (Wegerich) | 50 bis 400ml |
| Aloeextrakt | 200 bis 600ml. |

6. Kräutermischung nach Anspruch 3, ***dadurch gekennzeichnet,* dass** sich die Mischung aus folgenden Anteilen zusammensetzt:
| | |
|---|---|
| Calendula-Tinktur | 100 bis 600ml |
| Eukalyptus-Tinktur | 50 bis 200ml |
| Succus der Plantago Major (Wegerich) | 50 bis 400ml |
| Aloeextrakt | 200 bis 600ml |
| Vitaminisierter Hagebutte-Sirup | 200 bis 600ml. |

7. Kräutermischung nach Anspruch 2, ***dadurch gekennzeichnet,* dass** sich die Mischung aus folgenden Anteilen zusammensetzt:
| | |
|---|---|
| Calendula-Blumen | 50g |
| Folium des Eukalyptus | 25g |
| Folium der Banane | 25g. |

8. Kräutermischung nach Anspruch 1, ***dadurch gekennzeichnet,* dass** sich 100ml der Mischung in alkoholischem Extrakt aus folgenden Anteilen zusammensetzen:
| | |
|---|---|
| Calendula-Tinktur | 70ml |
| Eukalyptus-Tinktur | 30ml. |

9. Kräutermischung nach Anspruch 1, ***dadurch gekennzeichnet,* dass** sich 100ml der Mischung in hydroalkoholischer Suspension aus folgenden Anteilen zusammensetzen:
| | |
|---|---|
| Calendula-Tinktur | 50ml |
| Eukalyptus-Tinktur | 50ml. |

10. Kräutermischung nach einem der vorangehenden Ansprüche, ***dadurch gekennzeichnet,* dass** die Mischung Additive enthält, die wahlweise aus Stabilisatoren, Antibackmitteln, Verdickungsmittel, Konservierungsmittel und Färbungsmittel bestehen.

## Revendications

1. Composition d'herbes pour l'utilisation dans le traitement ou la guérison de maladies telles que déficience immunitaire virale, infections HIV, SIDA, hépatite virale, tuberculose, méningite et d'autres maladies virales ou d'étiologie microbienne chez les enfants et les adultes, contenant au moins de la feuille ou de la teinture de souci et de la feuille ou de la teinture d'eucalyptus.

2. Composition d'herbes selon la revendication 1, contenant de surcroît de suc de plantain.

3. Composition d'herbes selon l'une des revendications précédentes, contenant de surcroît de l'extrait d'aloès liquide.

4. Composition d'herbes selon l'une des revendications précédentes, contenant de surcroît du sirop d'églantine vitaminé.

5. Composition d'herbes selon la revendication 3, contenant les substances dans le rapport suivant :
| | |
|---|---|
| teinture de souci | 100 à 600 ml |
| teinture d'eucalyptus | 50 à 200 ml |
| suc de plantain | 50 à 400 ml |
| extrait d'aloès liquide | 200 à 600 ml. |

6. Composition d'herbes selon la revendication 4, contenant les substances dans le rapport suivant :
| | |
|---|---|
| teinture de souci | 100 à 600 ml |
| teinture d'eucalyptus | 50 à 200 ml |
| suc de plantain | 50 à 400 ml |
| extrait d'aloès liquide | 50 à 600 ml |
| sirop d'églantine vitaminé | 200 à 600 ml. |

7. Composition d'herbes selon la revendication 2 sous forme poudreuse ou pâteuse, contenant les substances dans le rapport suivant :
| | |
|---|---|
| fleurs de souci | 50 grammes |
| feuille d'eucalyptus | 25 grammes |
| feuille de plantain | 25 grammes. |

8. Composition d'herbes selon la revendication 1, dans laquelle 100 ml de composition sous forme d'extrait d'alcool contiennent les substances dans le rapport suivant :
| | |
|---|---|
| teinture de souci | 70 ml |
| teinture d'eucalyptus | 30 ml. |

9. Composition d'herbes selon la revendication 1, dans laquelle 100 ml de composition en suspension hydro-alcoolique contiennent les substances dans le rapport suivant :
| | |
|---|---|
| teinture de souci | 50 ml |
| teinture d'eucalyptus | 50 ml. |

10. Composition d'herbes selon l'une des revendications précédentes, contenant des additifs choisis parmi des stabilisants, des agents anti-coagulants, des agents épaississants, des agents de conservation et des agents de coloration.
